# EUROPEAN PATENT APPLICATION

(11) **EP 4 474 470 A1**
(43) Date of publication of application: **11.12.2024**
(21) Application number: 23305927.8
(22) Date of filing: 10.06.2023
(51) Int. Cl.: C12M 1/107, C12M 1/00, C12P 5/02

(54) **REACTOR AND METHOD FOR EX-SITU BIOLOGICAL METHANATION**

(71) Applicant: Université de Lorraine, 54000 Nancy (FR); Centre National de la Recherche Scientifique, 75016 Paris (FR)
(72) Inventor: HENRY, Guillaume, 54760 FAULX (FR); DELAUNAY, Stéphane, 54530 PAGNY-SUR-MOSELLE (FR); PACAUD, Stéphane, 35310 CINTRE (FR)
(74) Representative: IPAZ

(57) **Abstract**

The invention relates to Reactor for ex-situ biological methanation comprising a sealed tank intended to receive a liquid reaction medium, said reaction medium comprises a nutrient solution and a microbiological consortium, an external circulation circuit for the reaction medium, means for circulating the reaction medium, in a closed loop comprising the sealed tank and the external circulation circuit, from the sealed tank through the external circulation circuit to the sealed tank, a gas injection unit mounted on the external circulation circuit and arranged so that the reaction medium circulates through said gas injection unit and means for regulating the temperature of the reaction medium between 37 and 60°C; said gas injection unit comprises at least one gas/liquid membrane contactor, said at least one gas/liquid membrane contactor comprises a membrane intended to be interposed between a gas comprising gaseous dihydrogen H₂ and the reaction medium circulating through the gas injection unit and said at least one gas/liquid membrane contactor has a pore size comprised between 2 and 100 nm and being arranged to dissolved gaseous H₂ in the reaction medium, circulating through the gas injection unit, by diffusing gaseous H₂, through the pores of the membrane, from one side to another side of the membrane.

## Description

### Field of the invention

The present invention relates to methanation process and, in particular, to biological methanation.

Methanation is a chemical, or biological, reaction leading to methane CH₄ synthesis from dihydrogen Hz and carbon monoxide CO or carbon dioxide COz in the presence of a catalyst.

### Background to the invention

In the field of methanation, current developments are focused on chemical "reactor-exchanger" type that optimizes the synergy between chemical reaction control and the heat exchange and on the type of catalyst, in particular noble metals, used to catalyze methanation.

Most of the efforts are focused on methanisation reactors. Methanation is distinct from methanisation. During methanisation process, the methanation is one of the chain reactions, which is implemented in-situ in the methanisation reactor. Methanisation is a complex process that involves several interdependent biological reactions that have different optimal operating conditions..

In methanisation reactor, methanation is limited by the diffusion of Hz in the digestate which is a complex rheological medium. Thus, methanation yield, that is methane production, is limited. Moreover, Hz is an inhibitor for bacteria which indicates that the introduction of Hz in a methanisation reactor could possibly disturb the anaerobic digestion.

The methanation process also requires relatively high pressures, typically 10 to 20 bars in order to enhance the solubilization of H₂, precluding accessibility to most of the potential users, such as farmers or small companies.

Methanisation process leads to methane and carbon dioxide formation. Then, this biogas is used to produce heat and electricity or purified to obtain biomethane. Heat production is generally partially valorized, leading to a decrease of the global yield of the methanisation process, and biogas purification requires significant amount of energy.

### Summary of the invention

An object of the invention is to provide a device and a method for methane production which:
- limits dihydrogen overconsumption of the global process,
- enables the use of simple nutrient media,
- is suitable to any type of users,
- is operated at low pressure,
- enables an easy maintenance of the reactor,
- increases methanation yield, and/or
- avoid purification of the biogas containing methane produced by the bioreactor.

To this end, there is provided a reactor for ex-situ biological methanation, said reactor, comprising:
- a sealed tank intended to receive a liquid reaction medium, said reaction medium comprises a nutrient solution and a microbiological consortium,
- an external circulation circuit for the reaction medium,
- means for circulating the reaction medium, in a closed loop comprising or consisting in the sealed tank and the external circulation circuit, from the sealed tank through the external circulation circuit to the sealed tank,
- a gas injection unit, preferably at least one gas injection unit, mounted on the external circulation circuit and arranged so that the reaction medium circulates through said gas injection unit; said gas injection unit comprises at least one gas/liquid membrane contactor, said at least one gas/liquid membrane contactor comprises a membrane, said membrane is:
   ▪ intended to be interposed between a gas comprising gaseous dihydrogen H₂ and the reaction medium circulating through the gas injection unit,
   ▪ arranged to dissolved gaseous Hz in the reaction medium, circulating through the gas injection unit, by diffusing gaseous H₂, or the gas which comprises the gaseous H₂, through the pores of the membrane, from one side to another side of the membrane of the at least one gas/liquid membrane contactor of the gas injection unit,
- means for regulating the temperature of the reaction medium between 37 and 60°C.

Preferably, the membrane has a pore size comprised between 2 and 100 nm.

Preferably, according to the present description, it is understood by "interposed between" inserted between, placed between, positioned between or disposed between. Preferably, the membrane is intended to physically separate the gas comprising gaseous dihydrogen H₂ from the reaction medium.

Preferably, the reactor comprises means arranged for regulating the pH of the reaction medium. Preferably, the means for regulating the pH is arranged to maintain the pH between 5.5 and 8.

Preferably, the membrane is a ceramic membrane.

Preferably, the membrane has a pore size higher than or equal to 2 nm, more preferably to 3, even more preferably to 4, most preferably to 5, even most preferably to 6, particularly advantageously to 7, more particularly advantageously to 8, even more particularly advantageously from the last 9 and in the most preferred manner to 10 nm.

Preferably, the membrane has a pore size less than or equal to 100 nm, more preferably to 95, even more preferably to 90, most preferably to 85, even most preferably to 80, particularly advantageously to 75, more particularly advantageously to 70, even more particularly advantageously from the last 65, in a particularly preferred manner to 60 and in the most preferred manner to 50 nm.

Preferably, each gas/liquid membrane contactor comprises one or more membrane(s).

Preferably, the reaction medium comprises dissolved carbon dioxide COz and/or dissolved carbon monoxide CO.

Preferably, each of the two ends of the external circulation circuit communicate with and/or are connected to the sealed tank.

Preferably, one end of the external circulation circuit communicates with the bottom of the sealed tank and, preferably, the other end of the external circulation circuit communicates with a side or the top of the sealed tank. Preferably, the side of the sealed tank, with which the external circulation circuit communicates, is located between the bottom, or the middle, or from an area comprised between the bottom, or the middle, and the top of the sealed tank. Preferably, the side of the sealed tank, with which the external circulation circuit communicates, is intended to be located in area of the sealed tank immersed in the reaction medium.

Preferably, the means for circulating the reaction medium is/are arranged so that the reaction medium circulate from the side of the sealed tank, then through the external circulation circuit, towards the bottom of the sealed tank.

Preferably, the reactor does not comprise any means to filter the reaction medium. Preferably, the reactor does not comprise any means to filter the reaction medium along the closed loop.

Preferably, the at least one gas/liquid membrane contactor and/or the at least membrane of the at least one gas/liquid membrane contactor is a cross-flow filtration means.

The reactor may comprise means arranged to concentrate the microbiological consortium contained in the reaction medium. Preferably, the means arranged to concentrate the microbiological consortium is mounted on a further external circulation circuit. The means to concentrate the microbiological consortium may be dead cross-flow filtration means.

Preferably, the membrane of the gas/liquid membrane contactor of the gas injection unit is intended to be inserted between a gas and the reaction medium circulating through the gas injection unit.

Gaseous Hz may be contained in a gas, comprising other gaseous species, in contact with the membrane or the gas in contact with the membrane may consists in Hz.

Preferably, the reactor and/or the gas injection unit and/or the at least one gas/liquid membrane contactor and/or the membrane and/or the size of the pores of the membrane and/or the pressure of the reaction medium and/or the pressure of the gas is arranged to prevent the reaction medium to diffuse through the pores towards the gas.

Preferably, the gas comprising gaseous dihydrogen H₂ further comprises gaseous carbon dioxide.

Preferably, a first surface of the membrane, more preferably an outer surface of the membrane, with which the gas is intended to be in contact and/or a second surface of the membrane, more preferably an inner surface of the membrane, with which the reaction medium is intended to be in contact is uncoated.

Preferably, the membrane is pristine, that is as manufactured or as sold.

Preferably, the membrane has to two faces, sides or surfaces. Preferably, the membrane comprises the pores. Preferably, the pores of the membrane are extending from one of the two faces, sides or surfaces of the membrane to the other faces, sides or surfaces of the membrane. Preferably, one of the two faces, sides or surfaces of the membrane is intended to be in contact with the reaction medium and the other faces, sides or surfaces of the membrane is intended to be in contact with the gas.

Preferably, the membrane may comprise at least one outer surface, side or face and at least one inner surface, side or face.

Preferably, the outer surface, side or face of the membrane is intended to be in contact with the gas and the inner surface, side or face of the membrane is intended to be in contact with the reaction medium.

Preferably, the membrane or the first surface of the membrane with which the gas is intended to be in contact and/or a second surface of the membrane with which the reaction medium is intended to be in contact is hydrophilic.

Preferably, the membrane or the first surface of the membrane with which the gas is intended to be in contact and/or a second surface of the membrane with which the reaction medium is intended to be in contact is not hydrophobic.

The sealed tank may comprise the reaction medium.

Preferably, the microbiological consortium comprises, preferably comprises only, or consists in hydrogenotrophic archaea, preferably hydrogenotrophic methanogens archaea and/or carboxydotrophic archea.

Preferably, the microbiological consortium is not suitable for methanisation or cannot be used for methanisation. Nevertheless, the microbiological consortium may comprise a fraction, preferably, a small fraction of archea other than hydrogenotrophic methanogens archaea and/or carboxydotrophic archea, for instance methanogenic archea, and/or bacteria.

Preferably, the nutrient solution comprises, preferably comprises only, or consists in a hydrolysate.

Preferably, the nutrient solution comprises, preferably comprises only, or consists in yeast extract.

Preferably, the reactor comprises a further gas injection unit mounted on the external circulation circuit and arranged so that the reaction medium circulates through said further gas injection unit, said further gas injection unit comprises at least one gas/liquid membrane contactor, said at least one gas/liquid membrane contactor comprises a membrane:
- intended to be interposed between a gas comprising gaseous carbon dioxide COz and/or gaseous carbon monoxide CO and the reaction medium circulating through the further gas injection unit,
- having a pore size comprised between 2 and 100 nm and being arranged to dissolved gaseous CO2 and/or gaseous CO in the reaction medium, circulating through the further gas injection unit, by diffusing gaseous CO2 and/or gaseous CO, through the pores of the membrane, from one side to another side of the membrane.

The reactor may comprise at least one further gas injection unit.

Preferably, each gas injection unit of the reactor may be identical, that is exhibits or has the exact same technical features and the exact same arrangement. Thus, preferably, each feature of a considered gas injection unit and each feature of a considered gas/liquid membrane contactor and each feature of a considered membrane is transposable or applies to any other gas injection unit and gas/liquid membrane contactor and membrane.

Preferably, in particular, each membrane of each gas/liquid membrane contactor of each gas injection unit is intended to be interposed between a gas and the reaction medium circulating through the gas injection unit. Preferably, in particular, each membrane of each gas/liquid membrane contactor of each gas injection unit is:
▪ intended to be interposed between a gas and the reaction medium circulating through the gas injection unit,
▪ arranged to dissolved the gas or a part of the gas in the reaction medium, circulating through the gas injection unit, by diffusing said gas through the pores of the membrane, from one side to another side of the membrane of the at least one gas/liquid membrane contactor of the gas injection unit.

The gas injection units may be mounted in series along the external circulation circuit.

The reactor may comprise one, two or more gas injection units.

Preferably, each gas injection unit is intended to dissolve a different gas within the reaction medium.

COz and/or CO may be contained in a gas further comprising other gaseous species. COz and/or CO may be contained in a gas intended to be in contact with the membrane or the gas intended to be in contact with the membrane may consists in COz and/or CO.

Preferably, the reactor comprises means for controlling the pressure of the gas intended to be in contact with the at least one gas/liquid membrane contactor, preferably of the gas injection unit and/or of the further gas injection unit, and in particular with the membrane, said means being arranged to maintain, in the gas injection unit and in the at least one gas/liquid membrane contactor, a pressure of said gas higher than a pressure of the reaction medium.

Preferably, the mean for controlling the pressure of the gas intended to be in contact with the at least one gas/liquid membrane contactor is disposed in the gas injection unit, preferably in the at least one gas/liquid membrane contactor.

Preferably, the reactor comprises means for controlling the pressure of the gas intended to be in contact with the at least one gas/liquid membrane contactor, and in particular with the membrane, said means being arranged to maintain, in the gas injection unit and in the at least one gas/liquid membrane contactor, a pressure of said gas lower than a threshold pressure above which H₂ gas bubbles and/or CO2 gas bubbles and/or CO gas bubbles are formed into the reaction medium.

According to the invention, there is also provided a method for ex-situ anaerobic biological methanation of dissolved dihydrogen Hz by a microbiological consortium in presence of dissolved carbon dioxide COz and/or dissolved carbon monoxide CO, said microbiological consortium is contained in a liquid reaction medium further comprising a nutrient solution, and preferably dissolved Hz and dissolved COz and/or dissolved CO, said reaction medium being contained in a sealed tank of a reactor for ex-situ biological methanation, said method comprising the step consisting of:
- regulating the temperature of the reaction medium between 37 and 60°C,
- circulating the reaction medium in a closed loop comprising the sealed tank and an external closed circulation circuit of the reactor,
- dissolving gaseous dihydrogen Hz in the reaction medium circulating through a gas injection unit of the reactor, said gas injection unit:
   ▪ being mounted on the external circulation circuit,
   ▪ comprises at least one gas/liquid membrane contactor, said at least one gas/liquid membrane contactor comprises a membrane having a pore size comprised between 2 and 100 nm through which the gaseous Hz diffuses, through the pores of the membrane, from one side to another side of the membrane.

Preferably, the method comprises the step consisting in regulating the pH of the reaction medium, preferably between 5.5 and 8.

Preferably, the pH regulation of the reaction medium is carried out, by the pH regulation means, in the sealed tank.

Preferably, the temperature regulation of the reaction medium is carried out, by the temperature regulation means, in the sealed tank.

The method may comprise the step of injecting gaseous COz and/or gaseous CO directly in the sealed tank or in the external circulation circuit. Gaseous COz and/or gaseous CO may be comprised in the reaction medium.

The method may comprise the step of injecting reaction medium, periodically or when necessary, reaction medium in the sealed tank.

The method may comprise the step of agitating the reaction medium, preferably in the sealed tank.

Preferably, the liquid reaction medium comprises the microbiological consortium, the nutrient solution, dissolved Hz and dissolved COz and/or dissolved CO.

Preferably, the reaction medium circulates from the sealed tank, preferably from the bottom of the sealed tank, through the external circulation circuit to the sealed tank, preferably to the top of the sealed tank.

Preferably, the method comprises the step of dissolving gaseous COz and/or gaseous CO in the reaction medium circulating through a further gas injection unit of the reactor, said further gas injection unit:
- being mounted on the external circulation circuit,
- comprises at least one gas/liquid membrane contactor, said at least one gas/liquid membrane contactor comprises a membrane having a pore size comprised between 2 and 100 nm through which the gaseous COz and/or the gaseous CO diffuses, through the pores, from one side to another side of the membrane.

Preferably, the gaseous COz and/or the gaseous CO is contained in a biogas or in a biogas or the gaseous COz and/or the gaseous CO is a biogas.

Preferably, the gaseous COz comes from a methanisation reactor, preferably said methanisation reactor is not part of the reactor according to the invention.

Preferably, the method does not comprise any other biological reaction step than methanation. Preferably, the method does not comprise methanisation reaction or is not methanisation.

Preferably, the pressure inside the reactor and/or the pressure of the reaction medium within the closed loop is maintained at a pressure less than or equal to 2 bars.

Preferably, the pressure inside the reactor and inside the closed loop is the same. Preferably, the pressure of the reaction medium within the sealed tank and the pressure of the reaction medium within the closed loop is the same.

Preferably, the pressure inside the reactor and/or the pressure of the reaction medium within the closed loop is slightly above ambient pressure or atmospheric pressure, preferably less than 500 milli bars gauge pressure (mbarg).

Preferably, the reactor according to the invention is arranged, more preferably specifically arranged, to implement the method according to the invention. Thus, any feature of the reactor according to the invention may be transposed and/or integrated in the method according to the invention and vice versa.

### Brief description of the drawings

Further inventive objects, features and advantages will become apparent from the following detailed description of several embodiments of the invention with references to the drawings, in which:

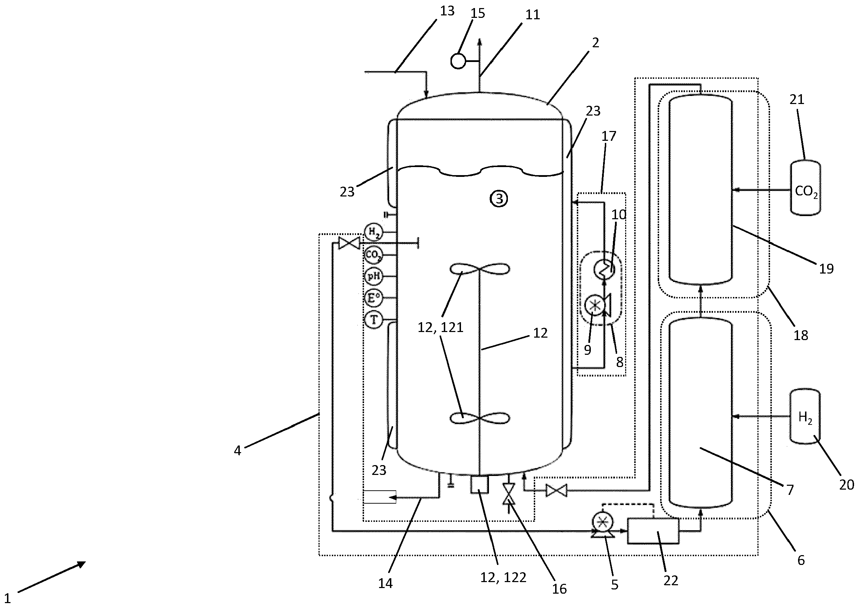

- FIGURE 1 is a schematic representation of an embodiment of the reactor for ex-situ biological methanation according to the invention.

### Detailed description of embodiments of the invention

The embodiments hereinafter described are not restrictive; other embodiments comprising a selection of features described hereinafter may be considered. A selection may comprise features isolated from a set of features (even if this selection is isolated among a sentence comprising other features thereof), if the selection is sufficient to confer a technical advantage or to distinguish the invention form the state of the art. This selection comprises at least a feature, preferably described by its technical function without structural features, or with a part of structural details if this part is sufficient to confer a technical advantage or to distinguish the invention form the state of the art on its own.

In reference to FIGURE 1, it is described an embodiment of a reactor for ex-situ biological methanation 1 according to the invention. The reactor 1 comprises a sealed tank 2. The sealed tank 2 is intended to receive a liquid reaction medium 3. The liquid reaction medium 3 comprises a nutrient solution and a microbiological consortium. As non-limiting indicative examples, the sealed tank 2 may exhibit a volume of 100 Liter and/or may be of the type of a column reactor. The sealed tank 2 exhibits a volume of 1.5 liter according to the embodiment.

The reactor 1 further comprises an external circulation circuit 4 for the reaction medium 3.

The reactor 1 further comprises means for circulating the reaction medium 3. The means for circulating the reaction medium 3 are arranged to circulate the reaction medium 3 in a closed loop 4, 2. The closed loop 4, 2 comprises the sealed tank 2 and the external circulation circuit 4. The means for circulating the reaction medium 3 and/or the sealed tank 2 and/or the external circulation circuit 4 are arranged to circulate the reaction medium 3 from the sealed tank 2 through the external circulation circuit 4 to the sealed tank 2. The means for circulating the reaction medium 3 comprises a volumetric pump 5 and a flowmeter 22 to feed the external circulation circuit 4 with a constant flow of reaction medium 3. According to the non-limiting embodiment, the flow of reaction medium 3 in the external circulation circuit 4 is set around at least 100 liters per hour (L/h), preferably at 500 L/h or more, more preferably 1 m³ L/h or more and most preferably 1.4 m³ L/h. The flow of reaction medium 3 in the external circulation circuit 4 is around 700 L/h according to the non-limiting embodiment.

The reactor 1 further comprises a gas injection unit 6 mounted on the external circulation circuit 4. The reactor 1 and/or the gas injection unit 6 is arranged so that the reaction medium 3 circulates through said gas injection unit 4. The gas injection unit 6 comprises at least one gas/liquid membrane contactor 7. The at least one gas/liquid membrane contactor 7 comprises a membrane intended to be interposed between a gas 20 comprising gaseous dihydrogen H₂ and the reaction medium 3 circulating through the gas injection unit 6. The membrane of the at least one gas/liquid membrane contactor 7 has a pore size comprised between 2 and 100 nm. The membrane is arranged to dissolved gaseous Hz contained in the gas 20 in the reaction medium 3, circulating through the gas injection unit 6, by diffusing gaseous H₂, through the pores of the membrane, from one side to another side of the membrane.

According to the non-limiting embodiment, the gas 20 injected in the gas injection unit 6 is a gas containing 80% of H₂ and 20% of COz in volume.

According to the invention, it is understood by "gas/liquid membrane contactor" any device comprising two compartments or chambers physically separated by a porous membrane. The reaction medium 3 flows in one of the compartments and is in contact with the membrane, the other compartment receives the gas 20, being in contact with the membrane, at a pressure higher than the pressure of the reaction medium 3.

Hz being is an inhibitor of the methanisation. In methanisation processes, the Hz quantity in the reaction medium has to be maintained low. Using a reactor for carrying out ex-situ methanation allows to increase H₂ quantity within the reaction medium by increasing the Hz partial pressure in the reaction medium.

Moreover, using an external circulation circuit 4 according to the invention to dissolve the Hz in the reaction medium 3 allows to:
- ease maintenance of the gas unit injection 6, and/or
- not disrupt and/or not interrupt the methanation process and/or avoid contamination of the reaction medium 3 during maintenance by isolating the sealed tank 2 from the gas unit injection 6.

The reactor 1 further comprises means for regulating the temperature 8 of the reaction medium 3 between 37 and 60°C. According to the non-limiting embodiment, the means for regulating the temperature 8 is arranged to regulate the temperature of the sealed tank 2 and so of the reaction medium 3 within the sealed tank 2. The means for regulating the temperature 8 comprises a pump 9 and a constant temperature bath 10. The means for regulating the temperature 8 is arranged for circulating a liquid medium, water according to the non-limiting embodiment, within a specific external circulation circuit 17 and within the constant temperature bath 10. The pump 9 and/or the specific external circulation circuit 17 is arranged for circulating the liquid medium regulated in temperature within a thermal sleeve 23 or thermal shell 23 or double shell 23 surrounding the sealed tank 2. Preferably, the means for regulating the temperature 8 and/or the constant temperature bath 10 is arranged to heat or cool the reaction medium 3.

The reactor 1 further comprises venting 11 for extracting the gas produced by methanation out of the sealed chamber 2. According to the invention, the gas produced by the microbiological consortium comprises biomethane.

The reactor 1 further comprises means 12 arranged to stir the reaction medium 3 within sealed tank 2. According to the embodiment, the means 12 comprises propellers 121 and an engine 122. The means 12 are arranged to achieve a vertical down pumping of the reaction medium 3 within the sealed tank 2. According to the non-limiting embodiment, the means 12 are arranged to stir the reaction medium 3 between 50 and 500 RPM.

The reactor 1 further comprises means 13 to feed, preferably continuously, the reaction medium 3 with nutrient solution. Preferably, the means 13 comprises controlling means arranged to independently control and/or adjust, preferably according to data measured from sensors of the reactor 1, nutrient solution and microbiological consortium feeding rate.

The reactor 1 further comprises sensors arranged to measure and/or monitor Hz, COz, pH, temperature, redox potential and/or a pressure sensor for the assessment of the level of liquid within the reaction medium 3 and/or an optical density sensor 15 arranged to measure/monitor the biomass concentration, that is micro-organism or microbial concentration or mass. According to the embodiment, and as a non-limiting example, the sensors are disposed within the sealed tank 2.

The reactor 1 further comprises flushing means 14 arranged to regularly remove some of the reaction medium 3 and maintain the level of reaction medium 3 constant in the sealed tank 2. Preferably, the flushing means 14 are arranged to remove some of the reaction medium 3 according to data measured by the sensors of the reactor 1 and/or at regular intervals.

The reactor 1 further comprises flushing mean 16 arranged for removing effluents/sewages from the sealed tank 2. The means 16 may be arranged to drain or empty the content of the reactor 1, that is the reaction medium 3 out of the reactor 1, for instance for maintenance needs.

The pressure within the sealed tank 2, and so the pressure of the reaction medium 3, is less than or equal to 4 bars, preferably to 3.5 bars, more preferably to 3, even more preferably to 2.75, most preferably to 2.5, even most preferably to 2.25, particularly advantageously to 2, more particularly advantageously to 1.75, even more particularly advantageously from the last 1.5 and in the most preferred manner to 1.25 bars. According to a preferred embodiment of the invention, the reaction medium 3 is at ambient pressure. Using atmospheric pressures of reaction medium 3 lower than 2 bars, or at atmospheric pressure, and so a pressure of the reaction medium 3 lower than 1.5 bars in the gas injection unit 6, enables and/or is required to allow or ensure gaseous Hz diffusion, through the pores of the membrane, while maintaining low pressure, typically lower than or equal to 2 bars, of the gas 20 containing gaseous Hz in the gas injection unit 6.

According to an improvement, the reactor 1 may comprise a further gas injection unit 18. The further gas injection unit 18 is mounted on the external circulation circuit 4. The further gas injection unit 18 is arranged so that the reaction medium 3 circulates through said further gas injection unit 18. The further gas injection unit 18 comprises at least one gas/liquid membrane contactor 19, a single gas/liquid membrane contactor 19 according to the embodiment. The least one gas/liquid membrane contactor 19 comprises a membrane intended to be interposed between a gas 21 comprising gaseous carbon dioxide COz and/or gaseous carbon monoxide CO and the reaction medium 3 circulating through the further gas injection unit 18. According to the embodiment, and in a generally preferred manner, the further gas injection unit 18, and each of its components, is identical to the gas injection unit 6 according to the invention.

In the case the reactor comprises the gas injection unit 6 and a further gas injection unit 18, the gas injection unit 6 is preferably dedicated to Hz dissolution (and so the gas 20 preferably contains only H₂) and the gas injection unit 18 is preferably dedicated to COz and/or CO dissolution (and so the gas 21 preferably contains only CO and/or COz). This allows an independent and complete control of the quantity of Hz and the quantity of COz and/or CO injected in the reaction medium 3.

It has to be noted that the rector 1 may not comprise the further gas injection 18. The COz and/or the CO may be injected, as for the described embodiment, together with the Hz through the gas injection unit 6. Alternatively, the COz and/or the CO may be directly injected in the sealed tank 2 or in another part of the reactor 1 without gas injection unit. For instance, The COz and/or the CO may be bubbled in the reaction medium 3 within the sealed tank 2.

In a preferred embodiment, the reactor 1 is coupled or associated with a methanisation reactor or a biological reactor producing biogas. In particular, the COz and/or CO and/or the biogas comprising COz and/or CO produce by the methanisation reactor or the biological reactor feeds the further gas injection unit 18.

According to the embodiment, and in a preferable manner, the reaction medium 3 containing dissolved Hz or saturated with dissolved Hz, and in a lesser extend with COz and/or CO, is injected, at the outlet of the external circulation circuit 4, in the bottom of the reactor 1 to maximize the residence time of the reaction medium 3 enriched/saturated with Hz. For the same purpose, the reaction medium 3 is drawn, at the inlet of the external circulation circuit 4, from the upper side of the sealed tank 2 so to draw the part off the reaction medium 3 with long residence time (and so low level of Hz remaining).

The reactor 1, the gas injection units 6 and/or the further gas injection unit 18 according to the embodiment, comprises means for controlling the pressure of the gas 20, 21 intended to be in contact with the at least one gas/liquid membrane contactor 7, 19. The means for controlling the pressure of the gas 20, 21 are arranged to maintain a pressure of the gas 20, 21 higher than the pressure of the reaction medium 3. The means for controlling the pressure of the gas 20, 21 may be arranged to control and/or set the pressure according to data measured by the sensors of the reactor 1. Concerning the pressure of Hz 20 in the gas injection unit 6, it is preferable to measure the residual content of Hz in the gas exiting the sealed reactor 1 through the venting 11. A sensor is arranged within the sealed tank 2 to assess if Hz is saturated or not in the reaction medium 3.

Nevertheless, means for controlling the pressure of the gas 20, 21 may be arranged to maintain a given value of pressure of the gas 20, 21 within the gas injection units 6 and/or the further gas injection unit 18 without any control and/or any feedback from any sensor.

According to the invention, the means for controlling the pressure of the gas 20, 21 intended to be in contact with the at least one gas/liquid membrane contactor 7, 19 may either be arranged to maintain and/or control the pressure of the gas 20, 21 in contact with the membrane at a given pressure value or may be arranged to maintain and/or control an injecting flowrate value of the gas 20, 21 in the gas injection unit 6, 18.

The means for controlling the pressure of the gas 20, 21 brought in contact with the at least one gas/liquid membrane contactor 7, 19, are arranged to maintain, in the gas injection unit 6, 18, a pressure of the gas 20, 21 lower than a threshold pressure. The threshold pressure is set so that no H₂ gas bubbles are formed into the reaction medium 3.

The threshold pressure depends on different factors among which the type of membrane, the size of the gas/liquid membrane contactor 7, 19 and operating conditions. According to the non-limiting embodiment, the threshold pressure is lower than or equal to 2 bars, preferably than 1.75, more preferably to 1.5, even more preferably to 1.25, most preferably to 1, even most preferably to 0.75, particularly advantageously to 0.5, more particularly advantageously to 0.25 and even more particularly advantageously to 0.1 bar and may be higher than or equal to 50 mbars bar, preferably to 100 mbars.

According to the non-limiting embodiment, the gas injection units 6, 18 comprise one gas/liquid membrane contactor 7, 19. The gas/liquid membrane contactor 7, 19 comprise at least one ceramic membrane. The ceramic membrane has pores size of 1 or respectively 15 kilo Daltons (kD) according to the non-limiting embodiment. The ceramic membrane has respectively 19 or 7 round channels with a hydraulic diameter respectively 3.5 or 6 mm. The length of the membrane can be 1178 mm or 590 mm. The membrane is not hydrophobic and used as sold. The membrane is a commercial membrane Inside Céram^{™} purchase from TAMI Industries

Thus, according to the invention, it is not the surface properties of the membrane of the gas/liquid membrane contactor 7, 19 that prevents the reaction medium 3 to pass through the membrane. According to the invention, it is the size of the pores of the membrane and/or the pressure of the gas (containing H₂ or COz and/or CO), which is higher than the pressure of the reaction medium 3, that prevents the reaction medium 3 to pass through the membrane. In particular, according to the invention, it is the combination of the size of the pores of the membrane together with the pressure of the gas (containing H2 or CO2 and/or CO), which is higher than the pressure of the reaction medium 3, that prevents the reaction medium 3 to pass through the membrane inside the gaz 20, 21.

In particular, according to the invention, a cross-flow filtration membrane is used for the membrane of the of the gas/liquid membrane contactor 7, 19. The cross-flow membrane is twisted from its initial purpose, which is to separate/withdraw or concentrate an object from a liquid media.

It has to be noted that the membrane with such a size of pores according to the invention allows using very low pressure of gas (containing H₂ or CO₂ and/or CO), typically lower than 2 bars and a pressure of 680 mbars according to the non-limiting embodiment. H₂ or gas containing Hz, and in a lesser extend CO and gas containing CO, requires specific security measures when gas 20, 21 are pressurized, typically at a pressure of 2 bars or more. Thus, the reactor 1 according to the invention can be installed everywhere.

Preferably, according to the invention, a ratio between the pressure of the gas 20, 21 in the gas injection units 6, 18 and the pressure of the reaction medium 3 in the gas injection units 6, 18 is higher than or equal to 1, more preferably to 1.25, even more preferably to 1.5, most preferably to 1.75, even most preferably to 2, particularly advantageously to 2.5, more particularly advantageously to 3.

Alternatively, according to the embodiment, a difference between the pressure of the gas 20, 21 in the gas injection units 6, 18 and the pressure of the reaction medium 3 in the gas injection units 6, 18, that is the transmembrane pressure, is equal or higher than 10 mbars, preferably to 20, more preferably to 30, even more preferably to 40, most preferably to 50, even most preferably to 75, particularly advantageously to 100, more particularly advantageously to 150 and even more particularly advantageously to 200 mbars. According to the non-limiting embodiment, the transmembrane pressure of the gas 20, 21 in the gas injection units 6, 18 and the pressure of the reaction medium 3 in the gas injection units 6, 18 and is around 180 mbars.

On the other hand, using very low pressure of gas (containing H₂ or CO₂ and/or CO) according to the invention precludes using high pressures of reaction medium 3, it is understood by high pressures of reaction medium 3 pressures equal or higher than 2 bars. The efficiency of the methanation reaction in the reactor 1 is therefore slightly reduce due to the low pressure of the reaction medium 3.

The gas injection units 6, 18 according to the invention and/or the threshold pressure and/or the pressure of the reaction medium 3 allows to optimize the Hz consumption without overfeeding the reaction medium 3 with Hz. Due to low solubility of H₂ in aqueous media, forming Hz bubbles in the reaction medium 3 will lead to waste H₂ that will be release from the reaction medium 3 in the sealed tank 2 and flushed through venting 11 of the reactor 11.

The gas injection units 6, 18 according to the invention are therefore arranged so that the whole reaction medium 3 is regularly brought in contact with the gas/liquid membrane contactor 7, 19 thus maintaining high level or saturation level of Hz within the reaction medium 3. Hz having a low solubility in water and the stoichiometry of the methanation reaction requiring four times more H₂ than dissolved carbon dioxide or monoxide, H₂ dissolution is a key factor of methanation.

According to the embodiment, it is also described a method for ex-situ anaerobic biological methanation of dissolved dihydrogen Hz. According to the non-limiting embodiment, the method is implemented in the reactor 1 previously described. The anaerobic biological methanation is carried out by a microbiological consortium in presence of dissolved carbon dioxide COz and/or dissolved carbon monoxide CO. The microbiological consortium is contained in a liquid reaction medium 3. The reaction medium 3 further comprising a nutrient solution. The reaction medium 3 is, at least in part, contained in the sealed tank 2 of the reactor for ex-situ biological methanation 1.

The method comprises the step consisting in:
- regulating the temperature of the reaction medium 3 between 37 and 60°C,
- circulating the reaction medium 3 in the closed loop 4, 2 comprising the sealed tank 2 and the external closed circulation circuit 4 of the reactor 1,
- dissolving gaseous dihydrogen Hz in the reaction medium 3 circulating through the gas injection unit 6 of the reactor 1.

The method according to the invention does not comprise any other biological reaction step than methanation. In particular, the method according to the invention does not comprise methanisation process.

According to the non-limiting embodiment, the method further comprises the step consisting in regulating the pH of the reaction medium at a pH comprised between 5.5 and 8.

Preferably, the microbiological consortium comprises hydrogenotrophic archaea and/or carboxydotrophic archea. The microbiological consortium consists in hydrogenotrophic archaea and carboxydotrophic archea according to the non-limiting embodiment.

In Methanisation processes, the complex rheology of the digestate prevents good and fast homogenization of the concentration of H₂ dissolved in the digestate. Therefore, there is strong gradients of dissolved H₂ within the digestate. The reaction medium according to the invention, in particular due to the nutrient solution used according to the invention, is simple, highly soluble, prevents clogging and exhibits a rheology very close to that of water. This enhances the transfer, the agitation and the homogenization of the reaction medium 3 within the reactor 1. Furthermore, diffusion and dissolution of H2 within the reaction medium 3 is also promoted. Circulation of the reaction medium 3 in the external circuit 4, 17 is also improved and enhanced.

The nutrient solution comprises a hydrolysate. The nutrient solution consists in hydrolysate according to the non-limiting embodiment. The hydrolysate comprises yeast extract. The nutrient solution consists in yeast extract according to the non-limiting embodiment. The yeast extract is contained in a solution at a concentration of 1 gram per liter. As non-limiting examples, the hydrolysate may comprise or consist in microbial hydrolysate, or meat or casein or soy-based hydrolysate.

The advantage of hydrolysates is that it is common, simple, in abundant availability and cheap. Therefore, due to the use of CO2 and/or CO as main or single carbon source, the microbiological consortium may comprise a small amount of archea other than hydrogenotrophic archaea and/or carboxydotrophic archea (it may comprise methanogenic archea for instance). Thus, according to the invention, the microbiological consortium is mixed and enriched in hydrogenotrophic archaea and also, preferably, in carboxydotrophic archea.

The enrichment in hydrogenotrophic archaea and also, preferably, in carboxydotrophic archea may be carried out in function of data measured by the sensors of the reactor 1 and/or at regular intervals, preferably according to a database or pre-calculated data.

The feeding of the reaction medium 3 by the nutrient solution may be carried out in function of data measured by the sensors of the reactor 1 and/or at regular intervals, preferably according to a database or pre-calculated data. The nutrient solution is required for the archaea to develop.

The temperature of the reaction medium 3 is maintained between 30 and 65°C. Preferably, the temperature of the reaction medium 3 is maintained either at mesophilic temperatures (temperatures preferably comprises between 36 and 40°C) or at thermophilic temperature (temperatures preferably comprises between 53 and 60°C) according to the microbiological consortium. The temperature of the reaction medium 3 is set at 39°C according to the non-limiting embodiment.

The invention is not restricted to embodiments described above and numerous adjustments may be made within the scope of the invention.

Thus, in combinable alternatives of previous embodiments:
- a first surface of the membrane with which the gas 20, 21 is intended to be in contact and/or a second surface of the membrane with which the reaction medium 3 is intended to be in contact is uncoated, and/or
- a first surface of the membrane with which the gas 20, 21 is intended to be in contact and a second surface of the membrane with which the reaction medium 3 is intended to be in contact is hydrophilic.

## Claims

1. Reactor for ex-situ biological methanation (1) comprising:
• a sealed tank (2) intended to receive a liquid reaction medium (3), said reaction medium comprises a nutrient solution and a microbiological consortium,
• an external circulation circuit (4) for the reaction medium,
• means for circulating the reaction medium, in a closed loop (2, 4) comprising the sealed tank and the external circulation circuit, from the sealed tank through the external circulation circuit to the sealed tank,
• a gas injection unit (6) mounted on the external circulation circuit and arranged so that the reaction medium circulates through said gas injection unit; said gas injection unit comprises at least one gas/liquid membrane contactor (7), said at least one gas/liquid membrane contactor comprises a membrane:
▪ intended to be interposed between a gas comprising gaseous dihydrogen Hz (20) and the reaction medium circulating through the gas injection unit,
▪ having a pore size comprised between 2 and 100 nm and being arranged to dissolved gaseous Hz in the reaction medium, circulating through the gas injection unit, by diffusing gaseous H₂, through the pores of the membrane, from one side to another side of the membrane,
• means for regulating the temperature (17) of the reaction medium between 37 and 60°C.

2. Reactor (1) according to claim 1, wherein a first surface of the membrane with which the gas (20, 21) is intended to be in contact and a second surface of the membrane with which the reaction medium is intended to be in contact is uncoated.

3. Reactor (1) according to claim 1, wherein a first surface of the membrane with which the gas (20, 21) is intended to be in contact and a second surface of the membrane with which the reaction medium is intended to be in contact is hydrophilic.

4. Reactor (1) according to any of claims 1 to 3, wherein the microbiological consortium comprises hydrogenotrophic archaea and/or carboxydotrophic archea.

5. Reactor (1) according to any of claims 1 to 4, wherein the nutrient solution comprises a hydrolysate.

6. Reactor (1) according to any of claims 1 to 5, wherein the nutrient solution comprises yeast extract.

7. Reactor (1) according to any of claims 1 to 6, comprising a further gas injection unit (18) mounted on the external circulation circuit (4) and arranged so that the reaction medium (3) circulates through said further gas injection unit, said further gas injection unit comprises at least one gas/liquid membrane contactor (19), said at least one gas/liquid membrane contactor comprises a membrane:
• intended to be interposed between a gas (21) comprising gaseous carbon dioxide COz and/or gaseous carbon monoxide CO and the reaction medium circulating through the further gas injection unit,
• having a pore size comprised between 2 and 100 nm and being arranged to dissolved gaseous COz and/or gaseous CO in the reaction medium, circulating through the further gas injection unit, by diffusing gaseous CO2 and/or gaseous CO, through the pores of the membrane, from one side to another side of the membrane.

8. Reactor (1) according to any of claims 1 to 7, comprising means for controlling the pressure of the gas (20, 21) intended to be in contact with the at least one gas/liquid membrane contactor (7, 19), said means being arranged to maintain a pressure of said gas higher than a pressure of the reaction medium (3).

9. Reactor (1) according to to any of claims 1 to 7, comprising means for controlling the pressure of the gas (20, 21) intended to be in contact with the at least one gas/liquid membrane contactor (7, 19), said means being arranged to maintain, in the gas injection unit (6, 18), a pressure of said gas lower than a threshold pressure above which H₂ gas bubbles are formed into the reaction medium (3).

10. Method for ex-situ anaerobic biological methanation of dissolved dihydrogen Hz by a microbiological consortium in presence of dissolved carbon dioxide COz and/or dissolved carbon monoxide CO, said microbiological consortium is contained in a liquid reaction medium (3) further comprising a nutrient solution, said reaction medium being contained in a sealed tank (2) of a reactor for ex-situ biological methanation (1), said method comprising the step consisting of:
• regulating the temperature of the reaction medium between 37 and 60°C,
• circulating the reaction medium in a closed loop (2, 4) comprising the sealed tank and an external closed circulation circuit (4) of the reactor,
• dissolving gaseous dihydrogen H₂ in the reaction medium circulating through a gas injection unit (6, 18) of the reactor, said gas injection unit:
▪ being mounted on the external circulation circuit,
▪ comprises at least one gas/liquid membrane, said at least one gas/liquid membrane contactor comprises a membrane having a pore size comprised between 2 and 100 nm through which the gaseous Hz diffuses, through the pores of the membrane, from one side to another side of the membrane.

11. Method according to the previous claim, further comprising the step of dissolving gaseous COz and/or gaseous CO in the reaction medium (3) circulating through a further gas injection unit (18) of the reactor (1), said further gas injection unit:
• being mounted on the external circulation circuit (4),
• comprises at least one gas/liquid membrane contactor (19), said at least one gas/liquid membrane contactor comprises a membrane having a pore size comprised between 2 and 100 nm through which the gaseous COz and/or the gaseous CO diffuses, through the pores, from one side to another side of the membrane.

12. Method according to the claim 10 or 11, wherein the gaseous COz and/or the gaseous CO is contained in a biogas.

13. Method according to any of claims 10 to 12, wherein the gaseous COz comes from a methanisation reactor.

14. Method according to any of claims 10 to 13, said method does not comprise any other biological reaction step than methanation.

15. Method according to any of claims 10 to 14, wherein the pressure inside the reactor is maintained at a pressure less than or equal to 2 bars.
